(19) Europäisches Patentamt
European Patent Office
Office européen des brevets

(11) **EP 0 573 347 B1**

(12) **FASCICULE DE BREVET EUROPEEN**

(45) Date de publication et mention
de la délivrance du brevet:
**26.02.1997 Bulletin 1997/09**

(51) Int. Cl.⁶: **B01J 29/18**, B01J 29/06

(21) Numéro de dépôt: **93401394.7**

(22) Date de dépôt: **01.06.1993**

(54) **L'utilisation d'un catalyseur à base de zéolite mordenite modifiée en isomérisation d'une coupe C8 aromatique**

Die Verwendung eines modifizierten Mordenit-Zeolith-Katalysators für die Isomerisierung einer aromatischen C8- Fraktion

Use of a modified mordenite zeolite catalyst for isomerizing an aromatic C8-fraction

(84) Etats contractants désignés:
**DE GB IT**

(30) Priorité: **03.06.1992 FR 9206819**

(43) Date de publication de la demande:
**08.12.1993 Bulletin 1993/49**

(73) Titulaire: **INSTITUT FRANCAIS DU PETROLE
92506 Rueil-Malmaison Cédex (FR)**

(72) Inventeurs:
• **Benazzi, Eric
F-78360 Montesson (FR)**
• **Travers, Christine
F-92500 Rueil Malmaison (FR)**
• **Joly, Jean-François
F-75300 Paris (FR)**
• **Bernhard, Jean-Yves
F-95140 Mennecy (FR)**

(56) Documents cités:
EP-A- 0 082 211          EP-A- 0 142 313
US-A- 3 933 983          US-A- 4 503 023
US-A- 4 753 910          US-A- 5 057 472

## Description

La présente invention concerne un procédé d'isomérisation d'une coupe C8 aromatique avec un catalyseur de type alumino-silicate comprenant une zéolithe MORDENITE dont on a modifié la sélectivité et/ou les propriétés catalytiques par désalumination de la surface externe de ses cristaux, et comprenant au moins un métal du groupe VIII de la classification périodique des éléments (Handbook of Chemistry and Physics, 65$^{ieme}$ édition, 1984-85), et une matrice. En général, la modification de la surface externe des cristaux de la zéolithe s'effectue par traitement de la mordénite par une solution d'un sel de l'acide fluorosilicique. De préférence, le sel choisi ne conduit pas à la formation de sels d'aluminium insolubles dans l'eau.

Actuellement, les catalyseurs utilisés industriellement dans les réactions d'isomérisation de coupes $C_8$ aromatiques sont essentiellement à base de zéolithe ZSM5, seule ou en mélange avec d'autres zéolithes, telles que la mordénite par exemple. Ces catalyseurs sont notamment décrits dans les brevets US-A-4467129 et US-A-4482773. L'intérêt de la zéolithe ZSM5 réside dans son excellente sélectivité de forme, qui conduit à une grande sélectivité en paraxylène, la sélectivité vis-à-vis des réactions secondaires indésirables de dismutation restant à un niveau très faible.

D'autres zéolithes à ouverture de pores plus large 12MR (ouverture à 12 atomes d'oxygène) ont aussi été utilisées, telle que la mordénite. Les catalyseurs à base de mordénite sont notamment décrits dans les brevets US-A-4723051 et US-A-4665258. Cependant, ces zéolithes ne possèdent pas de propriétés de sélectivité géométrique particulières. Ceci se traduit, quelque soit son rapport Si/Al, par des sélectivités en paraxylène plus faibles que celles obtenues pour la zéolithe ZSM5 et surtout par des productions de triméthylbenzènes très importantes. La production de triméthylbenzènes par dismutation est en effet favorisée dans la mordénite dont le système microporeux est plus ouvert que celui de la ZSM5: les ouvertures sont à 12 oxygènes au lieu de 10 pour la ZSM5.

Dans le cas des mordénites l'ajustement du rapport Si/Al à une valeur optimum pour la réaction à réaliser, nécessite dans la plupart des cas une étape de désalumination. En effet, le rapport Si/Al visé ne peut pas être dans tous les cas obtenu directement à la synthèse de la zéolithe (cas des rapports Si/Al>11).

Parmi les méthodes employées pour désaluminer les zéolithes, certaines font appel aux sels de l'acide hexafluorosilicique. Le brevet US-A-4503023 décrit la désalumination des zéolithes Y, Mordénite, Oméga, Rho, L, W, N-A, Offrétite, Clinoptilolite, Chabazzite et Erionite par traitement de ces dernières par des solutions d'hexafluorosilicate d'ammonium. Cette méthode permet d'atteindre, selon la nature de la zéolithe, un taux de désalumination au moins égal à 30%. Ce procédé permet d'une part de retirer des atomes d'aluminium de la charpente et de réinsérer à la place des atomes de silicium sous forme de tétraèdres de $SiO_4$ dans le réseau cristallin de la zéolithe qui conserve ainsi un taux de cristallinité élevé.

La demanderesse a découvert que, de façon surprenante, il est possible en réalisant des traitements de désalumination de la mordénite par des solutions diluées d'hexafluorosilicate d'ammonium, (0.0006 moles à 0.006 moles pour 100g de zéolithe sèche et de préférence entre 0.002 et 0.006 moles), d'obtenir des catalyseurs actifs et sélectifs pour la réaction d'isomérisation des $C_8$ aromatiques. Cette procédure de préparation nouvelle confère à la mordénite ainsi traitée, dont le taux de désalumination global est inférieur à 5% molaire, des propriétés de sélectivité très améliorées. Cela, se traduit par une inhibition surprenante des réactions secondaires indésirables telles que la réaction de dismutation. Cette nouvelle mordénite modifiée conduit par ailleurs à des sélectivités vis à vis des réactions parasites de désalkylation inférieures à celles des catalyseurs basés sur la ZSM5. Les solides ainsi obtenus présentent des performances en isomérisation des $C_8$ aromatiques non seulement meilleures que celles des mordénites de l'art antérieur mais également au moins équivalentes, voire supérieure, aux performances des catalyseurs à base de ZSM5.

La mordénite utilisée dans le catalyseur de la présente invention est fabriquée à partir d'une mordénite soit du type à petits pores soit du type à larges pores, aussi bien synthétisée en milieu hydroxyde que fluorure.

La mordénite du type à petits pores possède une teneur pondérale en sodium par rapport au poids de mordénite sèche généralement comprise entre 4 et 6.5%, un rapport atomique Si/Al global généralement compris entre 4 et 7, un volume de maille élémentaire généralement compris entre 2.76 et 2.80 nm$^3$ (avec 1 nm=10$^{-9}$m) et n'adsorbe habituellement que des molécules de diamètre cinétique inférieur à environ 4,4x10$^{-10}$m.

La mordénite du type à larges pores, synthétisée en milieu OH$^-$ ou bien en milieu F$^-$, par exemple selon le brevet EP-A-427579 correspondant au brevet CA-A-2 027301), se distingue de celle du type à petits pores par le fait qu'elle peut adsorber des molécules de diamètre cinétique supérieur à environ 6.6x10$^{-10}$m, donc en particulier des molécules de benzène, que son rapport atomique Si/Al global est généralement compris entre 4.5 et 20.

Le brevet EP-A-0082211 décrit l'augmentation du taux de silice dans les cristaux de la mordenite par traitement par une solution de sel d'acide fluorosilicique.

Avant de sélectiver la mordénite par traitement par des solutions de sels d'acide fluorosilicique, il peut être nécessaire d'amener le rapport Si/Al global de la mordénite à des valeurs supérieures à celles obtenues à la synthèse et mentionnées ci-avant, c'est à dire supérieure à un rapport Si/Al de 20 dans le cas des mordénites à larges pores et supérieur à 7 dans le cas des mordénites à petits pores. Afin d'obtenir des mordénites désaluminées dans une large gamme de rapport Si/Al, toute technique connue de l'homme du métier pourra être employée comme, par exemple, l'attaque acide directe, la calcination en présence ou non de vapeur d'eau de la forme NH$_4^+$ suivie d'une ou de plusieurs attaques acides, les cycles "calcination(s) - attaque(s) acide(s)". Dans le cas spécifique de la mordénite à petits pores, il faut s'assurer que les traitements retenus ont bien conduit à une ouverture des canaux.

La modification de la sélectivité des mordénites par traitement par des solutions diluées de fluorosilicates se fait sur des mordénites dont le rapport Si/Al est compris environ entre 4.5 et 100 et de préférence entre 4.5 et 15, lesdites mordénites sont sous forme Na, H, $NH_4$ ou sous toute forme mixte combinaisons des trois dernières, et de préférence sur la forme $NH_4$. La mise sous forme $NH_4$ s'effectue soit sur la mordénite brute de synthèse forme sodique sur laquelle plusieurs échanges ioniques par des solutions concentrées de nitrate d'ammonium (10N) permettent d'obtenir une teneur en pondérale en sodium par rapport au poids de mordénite sèche généralement inférieure à 2000 ppm, de préférence à 1000 ppm et, de manière encore plus préférée, à 500 ppm, soit sur les mordénites désaluminées, pour lesquelles plusieurs échanges successifs au nitrate d'ammonium permettent d'obtenir la forme $NH_4$ de ladite zéolithe.

Le fluorosilicate utilisé en tant qu'agent de désalumination et de source de silicium, permettant ainsi la réinsertion d'atomes de silicium au sein du réseau cristallin de la mordénite à la place des atomes d'aluminium extraits, peut être l'un des sels possédant la formule suivante: $M_{2/x}SiF_6$

où M est un cation métallique ou non métallique possédant la valence x. Les cations M peuvent donc être $NH_4^+$, des alkyls ammonium, $K^+$, $Na^+$, $Li^+$, $Ba^{2+}$, $Mg^{2+}$, $Cd^{2+}$, $Cu^+$, $Cu^{2+}$ $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Tl^+$ et $H^+$. De préférence, l'hexafluorosilicate d'ammonium est utilisé, car il conduit à la formation de sel d'aluminium $(NH_4)_3AlF_6$ soluble dans l'eau qui peut être ainsi aisément éliminé. En général, la température de traitement de la mordénite par l'hexafluorosilicate d'ammonium est comprise entre 20 et 100°C, et de préférence entre 50 et 100 °C. Les quantités d'hexafluorosilicate d'ammonium employées, inférieures à 0.006 moles pour 100g de mordénite sèche, sont calculées par rapport à une mordénite séchée sous flux d'air, à 450° C, durant 4 heures. Le traitement de la mordénite s'effectue en présence d'acétate d'ammonium qui permet de tamponner le pH du milieu réactionnel à des valeurs, comprises entre 4 et 8, et de préférence entre 5.5 et 7, valeurs de pH pour lesquelles la zéolithe ne subit pas de destruction de la charpente par attaque acide directe.

Après l'étape d'addition de la solution d'hexafluorosilicate d'ammonium à la suspension de mordénite dans une solution d'acétate d'ammonium, le mélange réactionnel est laissé, sous agitation vigoureuse, à la température désirée pendant une période comprise entre 30 minutes et 48 heures, mais de préférence comprise entre 1 et 2 heures.

La mordénite est ensuite filtrée à la température de la réaction et abondamment lavée à l'eau bouillante. Le volume d'eau bouillante utilisée pour effectuer ces lavages correspond à un v/p = 150 ml/g, (le rapport v/p est le rapport volume d'eau bouillante sur quantité de zéolithe sèche traitée).

Après ce traitement, la mordénite modifiée subit un traitement thermique destinée à décomposer les cations ammonium présents au sein du réseau et à obtenir ainsi la forme acide (H-M) de la mordénite.

La zéolithe peut ensuite être soumise au dépôt d'au moins un métal du groupe VIII de préférence choisi dans le groupe formé par le platine et le palladium, et mise en forme par toute technique connue de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière. La teneur en mordénite du mélange ainsi obtenu est généralement comprise entre 0.5 et 99.99% et avantageusement comprise entre 40 et 90% en poids par rapport au mélange (mordénite + matrice). Elle est plus particulièrement comprise entre environ 10 et 60% et, de préférence, entre environ 15 et 40% en poids par rapport au mélange (mordénite + matrice).

Dans la suite du texte on désignera par le terme support le mélange mordénite + matrice.

La mise en forme peut être réalisée avec d'autres matrices que l'alumine, telles que par exemple la magnésie, la silice alumine, les argiles naturelles (kaolin, bentonite) et par d'autres techniques que l'extrusion, telles que le pastillage ou la dragéification.

La métal hydrogénant du groupe VIII, de préférence Pt et /ou Pd, peut également être déposé sur le support par tout procédé connu de l'homme de l'art et permettant le dépôt du métal sur la mordénite. On peut utiliser la technique d'échange cationique avec compétition où le compétiteur est de préférence le nitrate d'ammonium, le rapport de compétition étant au moins égal à environ 50 et avantageusement d'environ 50 à 200. Dans le cas du platine ou du palladium, on utilise habituellement un complexe tétramine du platine ou un complexe tétramine du palladium : ces derniers se déposeront alors pratiquement en totalité sur la mordénite. Cette technique d'échange cationique peut également être utilisée pour déposer directement le métal sur la poudre de mordénite, avant son mélange éventuel avec une matrice.

Le dépôt du métal (ou des métaux) du groupe VIII est suivi en général d'une calcination sous air ou oxygène, usuellement entre 300 et 600°C durant 0.5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures ; de préférence on opèrera entre 350° et 550°C pendant 2 à 5 heures. La teneur en métal du groupe VIII (de préférence Pt et/ou Pd) déposé sur le catalyseur est obtenue à l'issue de l'échange est comprise habituellement entre 0.05 et 1.5%, de préférence entre 0.1 et 1%, en poids par rapport à l'ensemble du catalyseur.

On peut également déposer le platine et/ou le palladium non plus directement sur la mordénite, mais sur le liant aluminique, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. En général après le dépôt de platine et/ou de palladium, le catalyseur est comme précédemment soumis à une calcination puis réduit sous hydrogène comme indiqué ci-dessus.

Le catalyseur bifonctionnel obtenu par les procédures précédentes est mis en oeuvre dans les réactions d'isomérisation d'une coupe C8 aromatique, comprenant par exemple soit uniquement un mélange de xylènes, soit un mélange de xylène(s) et d'éthylbenzène.

L'isomérisation des alkyl-aromatiques, et en particulier des xylènes, revêt une importance commerciale considérable. C'est en général surtout le paraxylène qui est le produit le plus recherché, car il est notammant utilisé comme intermédiaire dans la fabrication des fibres polyesters. On préfère fabriquer le paraxylène en isomérisant le métaxylène, qui lui peut être obtenu par isomérisation de l'orthoxylène L'éthylbenzène qui est difficilement séparable par distillation du mélange des xylènes (les points d'ébullition des différents composés sont très proches), se trouve très souvent dans la charge d'isomérisation des hydrocarbures aromatiques en C8.

Les conditions opératoires du procédé d'isomérisation d'une coupe C8 aromatique effectuée en présence d'au moins un catalyseur selon l'invention sont les suivantes :

- température comprise entre 240 et 600°C, de préférence entre 350 et 510° C,
- presion comprise entre 0.05 et 10 MPa, de préférence entre 0.2 et 3 MPa,
- vitesse spatiale (pph, en masse de charge par unité de charge de catalyseur et par heure), comprise entre 0.5 et 200 $h^{-1}$, de préférence entre 2 et 100 $h^{-1}$,
- rapport molaire hydrogène sur hydrocarbure de la charge (H2/HC) compris entre 0.5 et 12, de préférence entre 2 et 6.

Les exemples qui suivent illustrent l'invention sans toutefois en limiter la portée ; ils sont donnés pour une charge formée de 80% d'orthoxylène et de 20% d'éthylbenzène (% en poids).

## Exemple 1: Catalyseur C1 conforme à l'invention

La matière première utilisée est une mordénite, qui possède un rapport Si/Al global de 5.2, un rapport Si/Al de charpente mesuré par infrarouge de 5.3, une teneur pondérale en sodium par rapport au poids de mordénite sèche d'environ 4.2%, un volume de maille élémentaire de 2.794 $nm^3$, un volume poreux à l'azote, mesuré à -196°C et à P/Po=0.19 de 0.163 $cm^3$ de liquide par gramme de mordénite et une surface spécifique, mesurée par la méthode B.E.T de 370 $m^2$/g.

La mordénite est tout d'abord soumise à 3 échanges ioniques dans une solution de NH4NO3 10N à environ 100°C pendant 4 heures, pour chaque échange. La teneur pondérale en sodium est ainsi inférieure à 50 ppm.

La mordénite forme $NH_4$ obtenue précédemment est ensuite soumise au traitement par l'hexafluorosilicate d'ammonium. Pour cela, 20 grammes de zéolithe sèche sont mis en suspension dans 200 ml d'une solution d'acétate d'ammonium (20 grammes d'acétate d'ammonium pour 200 ml d'eau distillée). Cette suspension est ensuite placée dans un tricol de 500 ml équipé d'un reflux, et d'une agitation mécanique. Le pH initial du milieu est de 7.05. La température est portée à 80°C. Puis à l'aide d'une pompe, 5 ml d'une solution d'hexafluorosilicate d'ammonium 0.2 M sont introduits à la vitesse de 15 ml/h par gramme de zéolithe sèche traitée, soit un débit de solution de 5 ml/min. En fin d'addition, la quantité d'hexafluorosilicate d'ammonium injectée représente 0.005 moles pour 100 gramme de zéolithe sèche. Le système est maintenu à la température de la réaction durant encore 2 heures. Puis, la solution est refroidie à l'ambiante, la valeur mesurée du pH en fin de réaction est de 5.9. Le solide est alors filtré et lavé avec un volume d'eau distillée bouillante au moins égal à 6 litres, c'est à dire au moins 300 ml d'eau distillée par gramme de zéolithe sèche (V/P=300 ml/g). La zéolithe ainsi traitée est séchée à l'étuve à 105°C durant une nuit, puis calcinée sous air sec de manière à déammoniaquer la mordénite et à obtenir la forme H. Le solide obtenu à l'issue des ces traitements est référencé HM1.

Ce dernier est ensuite intimement mélangé avec de l'alumine sur laquelle 0.3% poids de platine sont dispersés. Le catalyseur constitué par le mélange mordénite HM1+ alumine contient 40% en poids d'alumine. La teneur pondérale en platine du catalyseur final (contenant HM1) est donc d'environ 0.12%.

Le catalyseur ainsi fabriqué est ensuite mis en forme par pastillage, calciné sous air à 550°C durant 2 heures et réduit sous hydrogène à 500°C pendant 3 heures.

Le catalyseur C1 est alors testé en isomérisation du mélange orthoxylène (80% poids) et éthylbenzène (20% poids), à une température de 410°C, sous une pression de 1,2 MPa, avec une vitesse spatiale (pph) de 10 (heure)-1 et un rapport molaire hydrogène sur hydrocarbures (H2/HC) de 4 environ.

Les performances du catalyseur C1 (et des catalyseurs préparés dans les exemples suivants ), reportées dans le tableau I, sont définies par :

$$\text{Conversion de l'o-xylène (\%)} = \frac{\text{masse d'o-xylène dans la charge - masse d'o-xylène dans la recette}}{\text{masse d'o-xylène dans la charge}} \times 100$$

$$\text{Sélectivité en isomérisation (\%)} = \frac{\text{masse de m-xylène + masse de p-xylène}}{\text{masse de produits}} \times 100$$

Approche à l'équilibre de l'o-xylène (AEQ-ox) (%) = (nombre de moles d'o-xylène dans la recette/nombre de moles d'o-xylène à l'équilibre thermodynamique)x100

$$\text{Rendement en C8 aromatique (\%)} = \frac{\text{masse de C8 aromatiques et de naphtènes dans la recette}}{\text{masse totale de C8 aromatiques dans la charge}} \times 100$$

$$\text{Sélectivity en dismutation (\%)} = \frac{\text{masse de toluène + masse de triméthylbenzène + masse de benzène}}{\text{masse des produits}} \times 100$$

Séléctivité en dismutation (%) =

\F (masse de triméthylbenzènes + masse de toluène + masse de benzène; masse de benzène)

$$\text{Sélectivité en craquage (\%)} = \frac{\text{masse des gaz de C1 à C4}}{\text{masse des produits}} \times 100$$

## Exemple 2 : Catalyseur C2 conforme à l'invention

La matière première utilisée est la même mordénite, forme NH4, que celle utilisée dans l'exemple 1. Elle possède donc un rapport Si/Al=5.25 et une teneur pondérale en sodium inférieure à 50 ppm. Puis, dans cet exemple, la mordénite subit 5 traitements successifs à l'hexafluorosilicate identiques à celui décrit dans l'exemple 1. Après, chaque traitement à l'hexafluorosilicate d'ammonium un lavage à l'eau distillée bouillante est réalisée (v/p=300 ml/g). La zéolithe ainsi traitée est séchée à l'étuve à 105°C durant une nuit, puis calcinée sous air sec de manière à déammoniaquer la mordénite et à obtenir la forme H. Le solide obtenu à l'issue des ces traitements est référencé HM2.

Les étapes de mélange de la mordénite et de l'alumine, de dispersion du platine, de mise en forme, de réduction du catalyseur et les catalyseurs et les conditions de test d'isomérisation sont identiques à celles décrites dans l'exemple 1.

Les performances du catalyseur C2 ainsi obtenu (dont la teneur en platine est d'environ 0.12%) sont reportées dans le tableau 1.

## Exemple 3 : Catalyseur C3 conforme à l'invention

La matière première utilisée est une mordénite, qui possède un rapport Si/Al global de 10.5, un rapport Si/Al de charpente mesuré par infrarouge de 11.2, une teneur pondérale en sodium par rapport au poids de mordénite sèche d'environ 3,8%, un volume de maille élémentaire de 2755 $nm^3$, un volume poreux à l'azote, mesuré à -196°C et à P/Po=0.19 de 0.21 $cm^3$ de liquide par gramme de mordénite et une surface spécifique, mesurée par la méthode B.E.T de 480 $m^2$/g.

La mordénite est tout d'abord soumise à 3 échanges ioniques dans une solution de $NH_4NO_3$ 10N à environ 100°C pendant 4 heures, pour chaque échange. La teneur pondérale en sodium est ainsi inférieure à 50 ppm.

La mordénite forme $NH_4$ obtenue précédemment est ensuite soumise au traitement par l'hexafluorosilicate d'ammonium. Pour cela, 20 grammes de zéolithe sèche sont mis en suspension dans 200 ml d'une solution d'acétate d'ammonium (20 grammes d'acétate d'ammonium pour 200 ml d'eau distillée). Cette suspension est ensuite placée dans un tricol de 500 ml équipé d'un reflux, et d'une agitation mécanique Le pH initial du milieu est de 6.9. La température est portée à 80°C. Puis à l'aide d'une pompe, 5 ml d'une solution d'hexafluorosilicate d'ammonium 0.2 M sont introduits à la vitesse de 15 ml/h par gramme de zéolithe sèche traitée, soit un débit de solution de 5 ml/min. En fin d'addition, la quantité d'hexafluorosilicate d'ammonium injecté représente 0.005 moles pour 100 gramme de zéolithe sèche. Le système est maintenu à la température de la réaction durant encore 2 heures. Puis, la solution est refroidie à l'ambiante, la valeur mesurée du pH en fin de réaction est de 5.7. Le solide est alors filtré et lavé avec un volume d'eau distillée bouillante au moins égal à 6 litres, c'est à dire au moins 300 ml d'eau distillée par gramme de zéolithe sèche (V/P=300 ml/g). La zéolithe ainsi traitée est séchée à l'étuve à 105°C durant une nuit, puis calcinée sous air sec de manière à déammoniaquer la mordénite et à obtenir la forme H. Le solide obtenu à l'issue des ces traitements est référencé HM3.

Les étapes de mélange de la mordénite et de l'alumine, de dispersion du platine, de mise en forme, de réduction du catalyseur et les catalyseur et les conditions de test d'isomérisation sont identiques à celles décrites dans l'exemple 1.

Les performances du catalyseurs C3 ainsi obtenu (dont la teneur en platine est d'environ 0.12%) sont reportées dans le tableau 1.

### Exemple 4 : Catalyseur C4 conforme à l'invention

La matière première utilisée dans cet exemple est la même mordénite, forme NH4, que celle utilisée dans l'exemple 3. Elle possède donc un rapport global Si/Al= 10.5 et une teneur pondérale en sodium inférieure à 50 ppm. Puis, dans cet exemple, la mordénite subit 5 traitements successifs à l'hexafluorosilicate identiques à celui décrit dans l'exemple 3. Après, chaque traitement à l'hexafluorosilicate d'ammonium un lavage à l'eau distillée bouillante est réalisée (v/p=300 ml/g). En fin de traitement la zéolithe ainsi traitée est séchée à l'étuve à 105°C durant une nuit, puis calcinée sous air sec de manière à déammoniaquer la mordénite et à obtenir la forme H. Le solide obtenu à l'issue des ces traitements est référencé HM4.

Les étapes de mélange de la mordénite et de l'alumine, de dispersion du platine, de mise en forme, de réduction du catalyseur et les catalyseur et les conditions de test d'isomérisation sont identiques à celles décrites dans l'exemple 1.

Les performances du catalyseurs C4 ainsi obtenu (dont la teneur en platine est d'environ 0.12%) sont reportées dans le tableau 1.

### Exemple 5 : Catalyseur C5 non conforme à l'invention

Le catalyseur C5 contient la mordénite, forme H, de rapport Si/Al global de 5.25 utilisée lors de la préparation des catalyseurs C1 et C2. Mais, aucun traitement à l'hexafluorosilicate d'ammonium n'est réalisé dans cet exemple.

Les étapes de mélange de la mordénite et de l'alumine, de dispersion du platine, de mise en forme, de réduction du catalyseur et les catalyseurs et les conditions de test d'isomérisation sont identiques à celles décrites dans l'exemple 1.

Les performances du catalyseur C5 ainsi obtenu (dont la teneur en platine est d'environ 0.12%) sont reportées dans le tableau 1.

### Exemple 6 : Catalyseur C6 non conforme à l'invention

Le catalyseur C6 contient la mordénite, forme H, de rapport Si/Al global de 10.5 utilisée lors de la préparation des catalyseurs C3 et C4 Mais, aucun traitement à l'hexafluorosilicate d'ammonium n'est réalisé dans cet exemple.

Les étapes de mélange de la mordénite et de l'alumine, de dispersion du platine, de mise en forme, de réduction du catalyseur et les catalyseur et les conditions de test d'isomérisation sont identiques à celles décrites dans l'exemple 1.

Les performances du catalyseur C6 ainsi obtenu (dont la teneur en platine est d'environ 0.12%) sont reportées dans le tableau 1.

### Effet des traitements des mordénites par l'hexafluorosilicate d'ammonium, sur les sélectivités à iso-approche à l'équilibre

Le tableau I décrit les performances des catalyseurs C1, C2, C3, C4, C5 et C6, préparés selon les modes opératoires décrits précédemment. L'effet, des traitements à l'hexafluorosilicate d'ammonium sur les sélectivités est particulièrement mis en évidence.

Les catalyseurs C1, C2, C3, C4, C5 et C6 ont été testés dans les conditions décrites précédemment à savoir l'isomérisation d'un mélange orthoxylène (80% poids) et éthylbenzène (20% poids), à une température de 410°C, sous une pression de 1,2 MPa et un rapprt molaire hydrogène sur hydrocarbure (H2/HC) de 4.

TABLEAU 1

| Effet des traitements à l'hexafluorosilicate d'ammonium sur les sélectivités à iso-approche à l'équilibre | | | | | | |
|---|---|---|---|---|---|---|
| Catalyseurs | C5 (comp.) | C1 | C2 | C6 (comp.) | C3 | C4 |
| Exemple | 5 | 1 | 2 | 6 | 3 | 4 |
| % AEQ o-xylène | 94.3 | 94.7 | 94.5 | 94.2 | 94.6 | 94.4 |
| % Rdt $C_8$ aromatiques + naphtènes | 92.0 | 92.9 | 94.0 | 79.3 | 82.1 | 84.2 |
| % Dismutation | 4.1 | 3.3 | 2.4 | 9.4 | 7.1 | 5.2 |
| % Déalkylation | 0.2 | 0.1 | 0.1 | 0.6 | 0.6 | 0.4 |
| % Craquage | 0.9 | 0.8 | 0.7 | 4.9 | 4.9 | 4.4 |

Les catalyseurs C1, C2, C3, C4 conformes à l'invention sont plus performants que les catalyseurs C5 et C6 de l'art antérieur. En effet, à iso approche à l'équilibre de l'o-xylène, le rendement en isomérisarion des C8 aromatiques + naphtènes obtenus sur les catalyseurs C1, C2, C3 et C4 est supérieur à celui des catalyseurs C5 et C6. D'autre part, dans le cas des mordénites sélectivées selon l'invention (catalyseurs C1, C2, C3 et C4) la réaction secondaire de dismutation conduisant à la formation de triméthylbenzènes est fortement inhibée par rapport à ce que l'on obtient en présence de mordénite non sélectivée (catalyseurs C5 et C6).

L'analyse par fluorescence X, des mordénites H-$M_1$ et H-$M_2$ traitées selon l'invention, montre que la valeur des rapports Si/Al globaux ainsi mesurés sont sensiblement identiques au rapport Si/Al global de la mordénite de départ H-$M_5$ La même observation est faite dans le cas des mordénites H-$M_3$ et H-$M_4$ traitées et de la mordénite de départ non traitée H-M6.

On pense que le procédé conduit à une désalumination sélective de la surface externe des cristallites de mordénite. L'élimination des sites acides externes conduirait ainsi à une diminution du taux de dismutation des xylènes.

**Revendications**

1. Procédé d'isomérisation d'une coupe C8 aromatique à une température comprise entre 240 et 600°C, une pression comprise entre 0,05 et 10MPa, une vitesse spatiale comprise entre 0,5 et 200 $h^{-1}$ et avec un rapport molaire H2/HC compris entre 0,5 et 12, ledit procédé étant conduit en présence d'un catalyseur comprenant une matrice, au moins un élément du GVIII et une zéolithe mordénite caractérisé en ce que la mordénite présente un taux de désalumination global, inférieur à 5% molaire obtenu par désalumination des cristaux de zéolithe mordénite sur leur surface externe par traitement avec au moins une solution d'un fluorosilicate d'un cation à raison de 0,0006 à 0,006 moles pour 100g de zéolithe sèche.

2. Procédé selon la revendication 1, caractérisé en ce que l'élément du GVIII est choisi dans le groupe formé par le platine et le palladium.

3. Procédé selon la revendication 1, caractérisé en ce que la zéolithe mordénite à traiter présente un rapport Si/Al compris entre 4,5 et 100.

4. Procédé selon la revendication 1, caractérisé en ce que le rapport Si/Al est compris entre 4,5 et 15.

5. Procédé selon l'une des revendications précédentes, caractérisé en ce que la zéolithe mordénite à traiter est sous la forme $H^+$, $NH_4^+$, $Na^+$ ou sous une forme mixte résultant de la combinaison des formes précédentes.

6. Procédé selon l'une des revendications précédentes, caractérisé en ce que le cation associé au fluorosilicate est choisi dans le groupe formé par $NH_4^+$, les alkyls ammonium, $K^+$, $Na^+$, $Li^+$, $Ba^{2+}$, $Mg^{2+}$, $Cd^{2+}$, $Cu^+$, $Cu^{++}$, $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Tl^+$, $H^+$.

7. Procédé selon l'une des revendications précédentes, caractérisé en ce que la zéolithe mordénite et l'hexafluorosilicate sont laissés en contact entre 0,5 et 48h.

**8.** Procédé selon les revendications précédentes, caractérisé en ce que la matrice est choisie dans le groupe formé par l'alumine, la magnésie, la silice alumine, les argiles naturelles.

## Claims

**1.** A process for the isomerisation of an aromatic $C_8$ cut at a temperature in the range 240°C to 600°C, at a pressure in the range 0.05 MPa to 10 MPa, a space velocity in the range 0.5 $h^{-1}$ to 200 $h^{-1}$ and with a $H_2$/HC molar ratio in the range 0.5 to 12, said process being carried out in the presence of a catalyst comprising a matrix, at least one element from group VIII and a mordenite zeolite, characterized in that the mordenite has a global degree of dealuminisation of less than 5 mole % obtained by dealuminising the external surface of mordenite zeolite crystals by treatment with at least one solution of a fluorosilicate of a cation in a proportion of 0.0006 to 0.006 moles per 100 g of dry zeolite.

**2.** A process according to claim 1, characterized in that the element from group VIII is selected from the group formed by platinum and palladium.

**3.** A process according to claim 1, characterized in that the mordenite zeolite to be treated has a Si/Al ratio which is in the range 4.5 to 100.

**4.** A process according to claim 1, characterized in that the Si/Al ratio is in the range 4.5 to 15.

**5.** A process according to any one of the preceding claims, characterized in that the mordenite zeolite to be treated is in its $H^+$, $NH_4^+$, or $Na^+$ form or in a mixed form resulting from a combination of those forms.

**6.** A process according to any one of the preceding claims, characterized in that the cation associated with the fluorosilicate is selected from the group formed by $NH_4^+$, an alkyl ammonium, $K^+$, $Na^+$, $Li^+$, $Ba^{2+}$, $Mg^{2+}$, $Cd^{2+}$, $Cu^+$, $Cu^{2+}$, $Ca^{2+}$, $Cs^+$, $Fe^{2+}$, $Co^{2+}$, $Pb^{2+}$, $Mn^{2+}$, $Rb^+$, $Ag^+$, $Sr^{2+}$, $Zn^{2+}$, $Ti^+$ and $H^+$.

**7.** A process according to any one of the preceding claims, characterized in that the mordenite zeolite and the hexafluorosilicate are left in contact for between 0.5 and 48 hours.

**8.** A process according to any one of the preceding claims, characterized in that the matrix is selected from the group formed by alumina, magnesia, silica alumina and natural clays.

## Patentansprüche

**1.** Verfahren zur Isomerisierung einer aromatischen C8-Fraktion bei einer Temperatur, die zwischen 240 und 600°C enthalten ist, einem Druck, der zwischen 0,05 und 10 MPa enthalten ist, einer Raumgeschwindigkeit, die zwischen 0,5 und 200 $h^{-1}$ enthalten ist und mit einem Molverhältnis H2/HC, das zwischen 0,5 und 12 enthalten ist, wobei das Verfahren in Gegenwart eines Katalysators durchgeführt wird, der eine Matrix, wenigstens ein Element der Gruppe VIII und einen Mordenit-Zeolith umfaßt, dadurch gekennzeichnet, daß der Mordenit einen Gesamtentaluminierungsgrad von unter 5 Mol.% zeigt, der durch Entaluminierung von Mordenit-Zeolith-Kristallen auf ihrer äußeren Oberfläche durch Behandeln mit wenigstens einer Lösung eines Fluorosilicates eines Kations im Verhältnis von 0,0006 bis 0,006 Mol pro 100 g trockenem Zeolith erhalten wird.

**2.** Verfahren nach Anspruch 1, dadurch gekennzeichnet, daß das Element der Gruppe VIII aus der Gruppe ausgewählt ist, die durch Platin und Palladium gebildet ist.

**3.** Verfahren nach Anspruch 1, dadurch gekennzeichnet, daß der zu behandelnde Mordenit-Zeolith ein Si/Al Verhältnis zeigt, daß zwischen 4,5 und 100 liegt.

**4.** Verfahren nach Anspruch 1, dadurch gekennzeichnet, daß das Si/Al Verhältnis zwischen 4,5 und 15 liegt.

**5.** Verfahren nach einem der vorhergehenden Ansprüche, dadurch gekennzeichnet, daß der zu behandelnde Mordenit-Zeolith in der $H^+$-, $NH_4^+$-, $Na^+$-Form oder in einer Mischform vorliegt, welche aus der Kombination der vorhergehenden Formen resultiert.

**6.** Verfahren nach einem der vorhergehenden Ansprüche, dadurch gekennzeichnet, daß das mit Fluorosilicat verbundene Kation aus der Gruppe ausgewählt ist, die durch $NH_4^+$, die Ammoniumalkyle, $K^+$, $Na^+$, $Li^+$, $Ba^{2+}$, $Mg^{2+}$, $Cd^{2+}$,

$Cu^+$, $Cu^{++}$, $Ca^{2+}$, $Cs^+$, $Fe^{2+}$, $Co^{2+}$,. $Pb^{2+}$, $Mn^{2+}$, $Rb^+$, $Ag^+$ $Sr^{2+}$, $Zn^{2+}$,$Ti^+$, $H^+$ ausgewählt ist.

7.  Verfahren nach einem der vorhergehenden Ansprüche, dadurch gekennzeichnet, daß der Mordenit-Zeolith und das Hexafluorosilicat zwischen 0,5 und 48 Stunden in Kontakt gelassen werden.

8.  Verfahren nach den vorhergehenden Ansprüchen, dadurch gekennzeichnet, daß die Matrix aus der Gruppe ausgewählt ist, die durch Aluminiumoxid, Magnesiumoxid, Siliciumoxid-Aluminiumoxid, die natürlichen Tone gebildet ist.